(19) Europäisches Patentamt
European Patent Office
Office européen des brevets

(11) Veröffentlichungsnummer : **0 095 677**
**B1**

# (12) EUROPÄISCHE PATENTSCHRIFT

(45) Veröffentlichungstag der Patentschrift : **18.06.86**

(51) Int. Cl.[4] : **C 07 D249/08**, A 01 N 43/64

(21) Anmeldenummer : **83104998.6**

(22) Anmeldetag : **20.05.83**

(54) **Triazolyl-ketonoxime und -dioxime, Verfahren zu ihrer Herstellung und ihre Verwendung als Pflanzenwachstumsregulatoren.**

(30) Priorität : **28.05.82 DE 3220183**

(43) Veröffentlichungstag der Anmeldung : **07.12.83 Patentblatt 83/49**

(45) Bekanntmachung des Hinweises auf die Patenterteilung : **18.06.86 Patentblatt 86/25**

(84) Benannte Vertragsstaaten : **AT BE CH DE FR GB IT LI NL**

(56) Entgegenhaltungen :
**EP-A- 0 009 740**
**GB-A- 1 533 706**

(73) Patentinhaber : **BASF Aktiengesellschaft**
**Carl-Bosch-Strasse 38**
**D-6700 Ludwigshafen (DE)**

(72) Erfinder : **Rentzea, Costin, Dr.**
**Neuenheimer Landstrasse 72**
**D-6900 Heidelberg (DE)**
Erfinder : **Spiegler, Wolfgang, Dr.**
**Amsterdamer Strasse 16**
**D-6700 Ludwigshafen (DE)**
Erfinder : **Jung, Johann, Dr. Dipl.-Landwirt**
**Hardenburgstrasse 19**
**D-6703 Limburgerhof (DE)**

**EP 0 095 677 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue Triazolyl-ketonoxime und -dioxime, Verfahren zu ihrer Herstellung, Mittel zur Regulierung des Pflanzenwachstums, die diese Verbindungen enthalten und Verfahren zur Regulierung des Pflanzenwachstums mit diesen Verbindungen.

Es ist bekannt, daß bestimmte 2-Halogenethyl-trialkylammoniumhalogenide pflanzenwachstumsregulierende Eigenschaften aufweisen (vgl. US-PS 3 156 554). So läßt sich mit Hilfe von (2-Chlorethyl)-trimethylammoniumchlorid das Pflanzenwachstum beeinflussen. Allerdings ist die Wirksamkeit dieses Stoffes, vor allem bei niedrigen Aufwandmengen nicht immer befriedigend.

Es ist ferner bekannt, 1-(4-Bromphenyl)-1-allyloxy-2-(1,2,4-triazolyl-(1)-ethan zur Regulierung des Pflanzenwachstums vor allem von Raps, Weizen, Hafer, Roggen und Gerste zu verwenden (DE-OS 26 50 831). Dessen Wirkung ist jedoch, vor allem bei niedrigen Aufwandmengen nicht immer befriedigend.

Es wurde nun gefunden, daß Verbindungen der Formel I

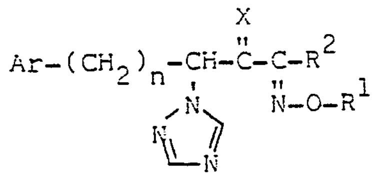

(I)

in der

Ar einen Arylrest aus der Gruppe Biphenylyl, Naphthyl oder Phenyl, der durch Halogenatome oder Trifluormethylreste oder durch Alkyl-, Alkoxy- oder Alkenylreste mit jeweils bis zu 5 C-Atomen und ferner durch einen Phenoxyrest substituiert sein kann, und

n 1 oder 2,

X Sauerstoff oder einen $=N-OR^3$-Rest bedeutet, wobei $R^3$ ein Wasserstoffatom, einen Alkyl-, Alkenyl- oder Alkinylrest mit jeweils bis zu 5 C-Atomen oder einen unsubstituierten oder durch Halogenatome, Trifluormethyl-, Alkyl- oder Alkoxyreste mit bis zu 4 C-Atomen substituierten Benzylrest oder einen $-CO-R^4$-Rest bedeutet, wobei $R^4$ einen Alkylrest mit bis zu 5 C-Atomen oder einen aromatischen Rest oder einen $-NH-R^5$-Rest bedeutet, wobei $R^5$ einen Alkylrest mit bis zu 4 C-Atomen oder einen aromatischen Rest bedeutet und

$R^1$ einen Alkylrest mit bis zu 8 C-Atomen, oder einen Phenylalkylrest bedeutet, und

$R^2$ einen Alkylrest mit bis zu 8 C-Atomen bedeutet,

sowie deren für Pflanzen verträgliche Salze und Metallkomplexe hervorragend zur Beeinflussung des Pflanzenwachstums geeignet sind und eine sehr gute Pflanzenverträglichkeit besitzen.

Die neuen Verbindungen der Formel I können sterisch in der Z- oder in der E-Form vorliegen. In den meisten Fällen liegen Gemische vor. Die Z- und E-Isomeren lassen sich bei einigen der erfindungsgemäßen Verbindungen beispielsweise durch Löslichkeitsunterschiede oder durch Säulenchromatographie trennen und in reiner Form isolieren. Diese werden von der vorliegenden Erfindung ebenfalls umfaßt. Als Mittel zur Beeinflussung des Pflanzenwachstums kann man sowohl die einheitlichen geometrischen Isomeren Z- und E wie auch deren bei der Synthese anfallenden Gemische verwenden. Aus wirtschaftlichen Gründen werden bevorzugt die letzteren verwendet, jedoch sind in manchen Fällen sterisch einheitliche Verbindungen mit einer günstigeren spezifischen Wirkung ausgestattet.

Ar bedeutet beispielsweise Biphenylyl, 1- und 2-Naphthyl, Phenyl, 2- und 4-Fluorphenyl, 3- und 4-Chlorphenyl, 4-Bromphenyl, 4-Methoxyphenyl, 4-Ethoxyphenyl, 4-Propoxyphenyl, 4-Methylphenyl, 2,4-Dimethylphenyl, 4-Ethylphenyl, 4-Propylphenyl, 4-n-Butylphenyl, 4-Isobutylphenyl, 4-tert.-butylphenyl, 4-Pentylphenyl, 4-Phenoxyphenyl, 3- und 4-Trifluormethylphenyl, 4-Allylphenyl, 3,4-Dichlorphenyl, 2,4-Dichlorphenyl und 2,6-Dichlorphenyl,

X bedeutet beispielsweise Sauerstoff oder einen $=N-O-R^3$-Rest;

$R^3$ bedeutet beispielsweise Wasserstoff, Methyl, Ethyl, 2-Methoxyethyl, n-Propyl, Isopropyl, n-Butyl, Isobutyl, Allyl, 2-Buten-1-yl, Pentenyl, Progargyl, Benzyl, 4-Fluorbenzyl, 4-Chlorbenzyl, 4-Trifluormethylbenzyl, 4-Brombenzyl, 4-Methylbenzyl, 4-tert.-Butylbenzyl, 2,4-Dichlorbenzyl, 3,4-Dichlorbenzyl und 2,6-Dichlorbenzyl.

$R^4$ bedeutet beispielsweise Methyl, Ethyl, n-Propyl, Isopropyl und Benzyl;

$R^5$ bedeutet beispielsweise Methyl, Ethyl, Propyl, n-Butyl, Phenyl oder 4-Chlorphenyl;

$R^1$ bedeutet beispielsweise Methyl, Ethyl, n-Propyl, Isopropyl, n-Butyl, Isobutyl, n-Pentyl, Isopentyl, n-Hexyl, n-Octyl, Benzyl und 2-Phenylethyl;

$R^2$ bedeutet beispielsweise Methyl, Ethyl, n-Propyl, Isopropyl, n-Butyl, Isobutyl, tert.-Butyl, n-Pentyl und n-Heptyl.

Triazolyl-ketonoxime und -dioxime der Formel I erhält man, wenn man ein Ketonoxim der Formel

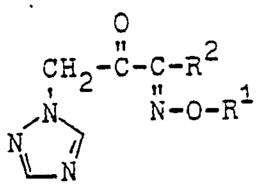

(II)

in der $R^1$ und $R^2$ die oben genannten Bedeutungen haben oder dessen Alkalienolat mit einem Arylalkylhalogenid der Formel

$$Ar—(CH_2)_n—Y \qquad (III)$$

in dem Ar und n die oben angegebenen Bedeutungen haben und Y Chlor, Brom oder lod bedeutet, umsetzt und die so erhaltenen Verbindungen der Formel I, in denen X zunächst ein Sauerstoffatom bedeutet, mit Hydroxylamin oder mit dessen Salzen zu Oximen der Formel I, in denen X eine =N—OH-Gruppe bedeutet, umsetzt und die so erhaltenen Dioxime mit Alkylierungsmitteln der Formel

$$R^3—Y \qquad (IV)$$

oder mit einem Säurechlorid der Formel $R^4—COCl$ oder mit einem Säureanhydrid der Formel $(R^4—CO)_2O$ oder ferner mit einem Isocyanat der Formel $R^5—N=C=O$ oder mit einem Carbamoylchlorid der Formel $R^5—NH—COCl$, in denen $R^3$, $R^4$ und $R^5$ die oben angegebenen Bedeutungen haben und Y Chlor, Brom oder lod bedeutet, umsetzt und die so erhaltenen Verbindungen — falls gewünscht — mit Säure in ihre Säureadditionssalze oder mit Metallsalzen in ihre Metallkomplexe überführt. Die Reihenfolge der Umsetzungen kann u. U. auch vertauscht werden.

Die Umsetzung von Ketonoximen (II) mit Arylalkylhalogeniden (III) verläuft gegebenenfalls in Gegenwart eines Lösungs- oder Verdünnungsmittels und vorzugsweise nach Zusatz einer starken anorganischen Base bei einer ausreichenden Temperatur i. a. unter 120 °C, bevorzugt zwischen 0 und 100 °C. Zu den bevorzugten Lösungsmitteln gehören Dimethylformamid, Diethylformamid, Dimethylacetamid, Diethylacetamid, N-Methylpyrrolidon, Hexamethyl-phosphorsäuretriamid, Dimethylsulfoxid und Sulfolan.

Geeignete Basen bzw. säurebindende Mittel sind beispielsweise Alkalihydride, wie Lithium-, Natrium- und Kaliumhydrid, Alkaliamide wie Natrium- und Kaliumamid oder Natrium- bzw. Kaliumdiisopropylamid, Alkalialkohole wie Natrium- oder Kaliummethoxid, -ethoxid oder tert.-butoxid, Lithium-, Natrium- oder Kalium-Triphenylmethyl und Naphthalin-Lithium, -Natrium oder Kalium.

Ketonoxime (II) sind ebenfalls neue Stoffe. Sie können, wie sich für den Fachmann bei Kenntnis ihrer Strukturformel ergibt, durch Umsetzung von bekannten 1-Halogen-3-oximino-3-alkylpropan-2-onen (V)

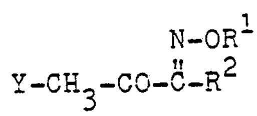

(V)

mit 1,2,4-Triazol erhalten werden. Eine geeignete Arbeitsvorschrift findet sich bei den Herstellungsbeispielen.

Die Umsetzung der Verbindungen der Formel I in denen X Sauerstoff bedeutet mit Hydroxylamin bzw. Hydroxylamin-Derivaten mit deren Salzen wird zweckmäßig in Wasser oder in einem mit Wasser mischbaren Lösungsmittel durchgeführt. Hierzu gehören beispielsweise Alkohole, wie Methanol, Ethanol, Propanol, Ethylenglykol, 2-Methoxyethanol ; Ether wie Tetrahydrofuran und Dioxan, Säureamide, wie Dimethylformamid, Diethylformamid, Dimethylacetamid, ferner N-Methyl-pyrrolidon und Hexamethylphosphorsäuretriamid oder Säuren wie Ameisensäure, Essigsäure und Propionsäure. Dabei ist es zweckmäßig, eine Base wie beispielsweise Natriumhydroxid, Kaliumhydroxid, Bariumhydroxid, Natriumacetat, Amine wie Triethylamin, Piperidin und N-Methylpiperidin oder eine Säure wie Salzsäure, Schwefelsäure, Phosphorsäure, Ameisensäure oder Essigsäure zuzusetzen.

Die Umsetzung verläuft im allgemeinen unterhalb von 80 °C, meist zwischen 0 und 40 °C.

Die Umsetzung der Triazolyl-dioxime der Formel I (X=N—OH) mit Alkylierungsmitteln, mit Säurechloriden und -anhydriden und mit Isocyanaten oder mit Carbamoylchloriden kann ohne Verdünnungsmittel oder in einem geeigneten Lösungsmittel stattfinden. Hierzu gehören beispielsweise Acetonitril ; Ether wie Diethyl-, Dipropyl-, Dibutyl- oder Methyl-tert.-butylether, Tetrahydrofuran, Dioxan und Dimethoxyethan ; Ester wie Essigsäureethylester ; Ketone wie Aceton und Methylethylketon ; chlorierte Kohlenwasserstoffe wie Methylenchlorid, Chloroform, Tetrachlorkohlenstoff, Dichlorethan, 1,1,1-Trichlorethan, Tetrachlorethan, Chlorbenzol ; Chlorbenzol ; Kohlenwasserstoffe wie Benzol, Toluol und Xylol. Dabei ist es zweckmäßig eine Base wie beispielsweise Alkali- und Erdalkalihydroxide wie Natriumhydroxid, Kaliumhydroxid, Calciumhydroxid, Bariumhydroxid, Alkali- und Erdalkalicarbonate wie Natriumcarbonat, Kaliumcarbonat, Natriumhydrogencarbonat, Kaliumhydrogencarbonat, Calciumcarbonat, Bariumcarbonat, Alkali- oder Erdalkalialkoholate wie Natriummethylat, Natriumethylat, Magnesiummethylat, Natriumisopropylat oder Kalium-tert.-butylat, Amine wie Triethylamin, N,N-Dimethylcyclohexylamin, Piperidin, N-Methylpiperidin oder Pyridin zuzusetzen.

Geeignete Reaktionsbeschleuniger sind beispielsweise Metallhalogenide wie Natriumbromid, Natriumiodid, Kaliumbromid, Kaliumiodid, tertiäre Amine wie 4-Dimethylaminopyridin oder 4-Pyrrolidinopyridin, Kronenether wie 12-Krone-4, 14-Krone-5, 18-Krone-6, Dibenzo-18-Krone-6 oder Dicyclohexano-18-krone-6 oder Azole wie Imidazol und 1,2,4-Triazol.

Als Phasentransferkatalysatoren können quaternäre Ammoniumsalze wie Tetrabutylammoniumbromid, -bromid, -iodid oder -hydrogensulfat, Benzyltriethylammoniumchlorid, Methyltrioctylammoniumchlorid und -bromid und Phosphoniumsalze wie Tetrabutylphosphoniumbromid und -iodid und Tetra-n-pentylphosphoniumbromid und -iodid dienen.

Die so erhaltenen Verbindungen der Formel I werden nach üblichen Methoden isoliert, gegebenenfalls gereinigt und gegebenenfalls mit Säuren oder Metallsalzen zu Salzen bzw. zu Metall-Komplexen umgesetzt.

Die folgenden Beispiele erläutern die Herstellung der neuen Verbindung :

## Beispiel 1

a) Herstellung eines Vorproduktes (V) :

Zu einer unter reinem Stickstoff gerührten Suspension von 68,2 g (0,75 Mol) Natrium-1,2,4-triazolid in 250 ml trockenem Tetrahydrofuran wird die Lösung von 144 g (0,74 Mol) 1-Brom-3-methoxyimino-butan-2-on (T. Wieland u. J. Stärke, Chem. Ber., *96*, 2410 (1963)) in 200 ml Tetrahydrofuran bei 25 °C in 2 Stunden zugetropft. Das Gemisch wird 2 Tage bei 25 °C und dann 3 Stunden bei 60 °C nachgerührt. Der entstandene Niederschlag wird abfiltriert und das Filtrat i. V. eingeengt. Man löst in 300 ml Methylenchlorid, wäscht dreimal mit je 50 ml Wasser, trocknet über Natriumsulfat und engt i. V. ein. Der Rückstand wird mit 80 ml Ether verrührt und über Nacht bei + 3 °C aufbewahrt, der Niederschlag abgesaugt, mit 30 ml kaltem (+ 5 °C) Ether, dann mit 100 ml n-Pentan gewaschen und anschließend getrocknet.

Es wurden 56 g (41 % d. Th.) 1-(1,2,4-Triazolyl-(1))-3-methoxyiminobutan-2-on als blaßgelbe Kristalle vom Schmp. 87 bis 89 °C erhalten.

b) Herstellung des Endproduktes

Zu einer unter reinem Stickstoff gerührten Suspension von 5 g (0,21 Mol) Natriumhydrid in 100 ml trockenem Dimethylformamid wird die Lösung von 36,4 g (0,2 Mol) 1-(1,2,4-Triazolyl-(1))-3-methoxyimino-butan-2-on in 50 ml Dimethylformamid bei 20 bis 25 °C zugetropft. Nach dreistündigem Nachrühren wird bei 25 °C die Lösung von 32,2 g (0,2 Mol) 4-Chlorbenzylchlorid in 35 ml Dimethylformamid zugetropft und weitere 15 Stunden nachgerührt. 40 ml Eiswasser werden vorsichtig zugetropft und das Gemisch i. V. eingeengt. Der Rückstand wird zwischen 300 ml Methylenchlorid und 80 ml Wasser verteilt, die organische Phase dreimal mit je 100 ml Wasser gewaschen, über $Na_2SO_4$ getrocknet und eingeengt. Der Rückstand wird mit 30 ml Ether verrührt und bei + 3 °C drei Stunden aufbewahrt. Der Niederschlag wird abgesaugt, mit 10 ml kaltem (+ 5 °C) Ether, dann mit 80 ml Petrolether gewaschen und getrocknet.

Es wurden 35,6 g (58 % d. Th.) 1-(4-Chlorphenyl)-2-(1,2,4-Triazolyl-(1))-4-methoxyimino-pentan-3-on als weiße Kristalle vom Schmelzpunkt 87 bis 88 °C erhalten.

## Beispiel 2

Zu einer Lösung von 7,6 g (0,11 Mol) Hydroxylaminhydrochlorid in 100 ml Ethanol werden 18 g (0,1 Mol) einer 30 %igen Natriummethylatlösung in Methanol bei 20 °C zugetropft. Anschließend wird die Lösung von 20 g des gemäß Beispiel 1 gewonnenen (0,07 Mol) 1-(4-Chlorphenyl)-2-(1,2,4-triazolyl-(1)-4-methoxyimino-pentan-3-on in 100 ml Ethanol zugegeben, das Gemisch 3 Tage bei 60 °C gerührt, dann auf —5 °C abgekühlt. Der Niederschlag wird abgesaugt, nacheinander mit 30 ml Eiswasser, mit 10 ml kaltem (+ 5 °C) Ethanol und mit 30 ml Ether gewaschen und getrocknet.

Es wurden 14,9 g (66,2 % d. Th.) 1-(4-Chlorphenyl)-2-(1,2,4-triazolyl-(1))-3-hydroxyimino-4-methoxyimino-pentan als farblose Kristalle vom Schmp. 162-164 °C erhalten.

## Beispiel 3

Eine Mischung aus 8 g (0,025 Mol) 1-(4-Chlorphenyl)-2-(1,2,4-triazolyl-(1))-3-hydroxyimino-4-methoxyimino-pentan, 0,5 g Imidazol und 50 ml Propionsäureanhydrid werden 12 Stunden bei 60 °C gerührt und anschließend i. V. eingeengt. Der Rückstand wird in 150 ml Ether gelöst und 30 Minuten mit 50 ml einer 6 %igen Natriumhydrogencarbonat-Lösung gerührt. Die organische Phase wird über Natriumsulfat getrocknet und i. V. — schließlich bis 70 °C und 0,2 mbar — eingeengt.

Es wurden 8,2 g (86,8 % d. Th.) 1-(4-Chlorphenyl)-2-(1,2,4-triazolyl-(1))-3-propionyloxyimino-4-methoxyimino-pentan als hellbraunes Harz erhalten.

IR (Film) : 2 980, 2 950, 1 773, 1 489, 1 362, 1 345, 1 272, 1 135, 1 048, 1 012, 895, 815 und 680 $cm^{-1}$.

Beispiel 4

Zu einer Lösung von 15,6 g (0,043 Mol) 1-(4-tert.-Butylphenyl)-2-(1,2,4-triazolyl-(1))-3-hydroxyimino-4-methoxyimino-pentan (vgl. Beispiel 78) in 100 ml Methanol werden 8,3 g (0,046 Mol) einer 30 %igen Natriummethylatlösung in Methanol bei 20 °C zugetropft. Anschließend wird die Lösung von 7,9 g (0,046) Benzylbromid in 15 ml Tetrahydrofuran zugegeben, das Gemisch 15 Stunden bei 20 °C gerührt und i. V. eingeengt. Der Rückstand wird zwischen 200 ml Methylenchlorid und 70 ml Wasser verteilt, die organische Phase dreimal mit je 50 ml Wasser gewaschen, über $NaSO_4$ getrocknet und eingeengt. Der Rückstand wird in 35 ml Petrolether gelöst, angeimpft und 16 Stunden im Kühlschrank aufbewahrt. Der entstandene weiße Niederschlag wird abgesaugt, mit 10 ml kaltem (— 5 °C) Petrolether gewaschen und getrocknet. Es wurden 16 g (85,7 % d. Th.) 1-(4-tert.-Butylphenyl)-2-(1,2,4-triazolyl-(1))-3-benzyloxyimino-4-methoxyimino-pentan vom Schmp. 108 bis 110 °C erhalten.

Durch entsprechende Abwandlung der vorstehenden Herstellbeispiele konnten die in der folgenden Tabelle aufgeführten Verbindungen erhalten werden.

(Siehe Tabelle Seite 6 ff.)

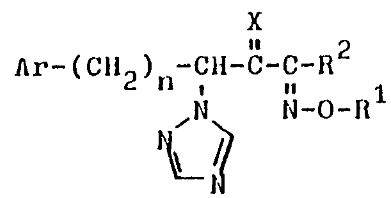

| Nr. | Ar | X | $R^1$ | $R^2$ | n | Phys. Konstante oder IR-Spektr. [$cm^{-1}$] (Film) |
|---|---|---|---|---|---|---|
| 5 | $4-BrC_6H_4-$ | O | $-CH_3$ | $-CH_3$ | 1 | Schmp. 107-109°C |
| 6 | " | =N-OH | " | " | 1 | Schmp. 170-172°C |
| 7 | " | $CH_3-NH-CO-O-N=$ | " | " | 1 | Harz |
| 8 | $C_6H_5-$ | O | " | " | 1 | Schmp. 70-72°C |
| 9 | " | =N-OH | " | " | 1 | Schmp. 140-142°C |
| 10 | " | O | $n-C_3H_7$ | " | 1 | 3030, 2965, 2878 1695, 1595, 1490 1268, 1200, 1130 1020, 750, 700 |
| 11 | " | O | " | " | 2 | 3028, 2964, 2880 1695, 1595, 1492 1358, 1270, 1130 1055, 1020, 750 |
| 12 | " | =N-OH | " | " | 1 | Schmp. 123-124°C |
| 13 | $1-C_{10}H_7$ | O | $-CH_3$ | " | 1 | Schmp. 138-140°C |
| 14 | " | =N-OH | " | " | 1 | Schmp. 198-200°C |

| Nr. | Ar | X | $R^1$ | $R^2$ | n | Phys. Konstante oder IR-Spektr. [$cm^{-1}$] (Film) |
|---|---|---|---|---|---|---|
| 15 | 1-$C_{10}H_7$ | 4-$FC_6H_4$-$CH_2$-O-N= | -$CH_3$ | -$CH_3$ | 1 | 3060, 2940, 1605 1511, 1276, 1224 1047, 1021, 1014 902, 825, 799, 781 |
| 16 | " | 2,6-$Cl_2C_6H_3$-$CH_2$-O-N= | " | " | 1 | Schmp. 122-132°C |
| 17 | 4-$FC_6H_4$ | O | " | " | 1 | Schmp. 93-95°C |
| 18 | " | =N-OH | " | " | 1 | Schmp. 141-143°C |
| 19 | " | $CH_2$=CH-$CH_2$-O-N= | " | " | 1 | 2940, 2822, 1605 1511, 1276, 1224 1139, 1053, 1023 985, 903, 679 |
| 20 | 3-$ClC_6H_4$- | O | " | " | 1 | 2976, 2940, 1695 1590, 1492, 1268 1130, 1045, 888 782, 690, 678 |
| 21 | " | =N-OH | " | " | 1 | Schmp. 136-138°C |
| 22 | 4-$ClC_6H_4$- | O | -$C_2H_5$ | " | 1 | Harz |
| 23 | " | =N-OH | " | " | 1 | Schmp. 161-162°C |
| 24 | " | 4-$ClC_6H_4$-$CH_2$-O-N= | " | " | 1 | 2982, 2940, 1494 1367, 1276, 1208 1139, 1092, 1048 1015, 956, 890 801 |

| Nr. | Ar | X | $R^1$ | $R^2$ | n | Phys. Konstante oder IR-Spektr. [$cm^{-1}$] (Film) |
|---|---|---|---|---|---|---|
| 25 | $4-ClC_6H_4$ | $4-BrC_6H_4-CH_2-O-N=$ | $-C_2H_5$ | $-CH_3$ | 1 | Harz |
| 26 | " | $4-(CF_3)C_6H_4-CH_2-O-N=$ | " | " | 1 | Harz |
| 27 | " | $n-C_4H_9-O-N=$ | " | " | 1 | Harz |
| 28 | " | $CH_3-O-CH_2CH_2-O-N=$ | " | " | 1 | Harz |
| 29 | " | O | $-C_3H_7-n$ | " | 1 | 2968, 2935, 1695 1590, 1484, 1268 1200, 1055, 1035 815, 678 |
| 30 | " | =N-OH | " | " | 1 | Schmp. 141-143°C |
| 31 | " | $CH_3-\overset{O}{\overset{\|}{C}}-O-N=$ | " | " | 1 | Harz |
| 32 | " | $Cl-CH_2-\overset{O}{\overset{\|}{C}}-O-N=$ | " | " | 1 | Harz |
| 33 | " | O | $-C_4H_9-n$ | " | 1 | 2970, 2880, 1698 1603, 1495, 1370 1278, 1140, 1035 880, 820, 678 |
| 34 | " | =N-OH | " | " | 1 | Schmp. 142-146°C |
| 35 | " | $CH_3-CH_2-\overset{O}{\overset{\|}{C}}-O-N=$ | " | " | 1 | Harz |

| Nr. | Ar | X | $R^1$ | $R^2$ | n | Phys. Konstante oder IR-Spektr. [$cm^{-1}$] (Film) |
|---|---|---|---|---|---|---|
| 36 | $4-ClC_6H_4-$ | O | $C_6H_5-CH_2-$ | $-CH_3$ | 1 | 3025, 2925, 1695 1590, 1480, 1355 1266, 1130, 1005 870, 812, 734 695 |
| 37 | " | =N-OH | " | " | 1 | Schmp. 184-186°C (Isomer I) |
| 38 | " | =N-OH | " | " | 1 | Schmp. 146-148°C (Isomer-Gemisch) |
| 39 | " | O | $-CH_3$ | $-C_2H_5$ | 1 | Schmp. 78-80°C |
| 40 | " | =N-OH | " | " | 1 | Schmp. 171-174°C |
| 41 | " | $CH_3-CH_2-C(=O)-O-N=$ | " | " | 1 | Harz |
| 42 | $3,4-Cl_2C_6H_3$ | O | " | $-CH_3$ | 1 | Schmp. 106-108°C |
| 43 | " | =N-OH | " | " | 1 | Schmp. 169-171°C |
| 44 | $2,4-Cl_2C_6H_3-$ | O | " | " | 1 | Schmp. 107-109°C |
| 45 | " | =N-OH | " | " | 1 | Schmp. 220-222°C (Isomer E) |
| 46 | " | =N-OH | " | " | 1 | Schmp. 158-160°C (Isomer Z) |

| Nr. | Ar | X | $R^1$ | $R^2$ | n | Phys. Konstante oder IR-Spektr. [$cm^{-1}$] (Film) |
|---|---|---|---|---|---|---|
| 47 | $2,4-Cl_2C_6H_3$ | $CH_3-CH_2-\overset{\overset{O}{\|}}{C}-O-N=$ | $-CH_3$ | $-CH_3$ | 1 | 2975, 2940, 1774 1580, 1496, 1466 1360, 1278, 1130 1100, 1045, 876 |
| 48 | " | $C_6H_5-\overset{\overset{O}{\|}}{C}-O-N=$ | " | " | 1 | Harz |
| 49 | " | O | $-C_2H_5$ | " | 1 | Schmp. 83-85°C |
| 50 | " | =N-OH | " | " | 1 | Schmp. 127-128°C |
| 51 | " | $CH_3-CH_2-\overset{\overset{O}{\|}}{C}-O-N=$ | " | " | 1 | Harz |
| 52 | " | O | $n-C_3H_7$ | " | 1 | 2960, 1692, 1580 1490, 1465, 1268 1035, 920, 862 825, 676 |
| 53 | " | =N-OH | " | " | 1 | Schmp. 131-134°C |
| 54 | " | $CH_3-CH_2-\overset{\overset{O}{\|}}{C}-O-N=$ | " | " | 1 | Harz |
| 55 | " | O | $-CH_3$ | $n-C_4H_9$ | 1 | 2960, 2937, 1707 1592, 1502, 1475 1276, 1138, 1104 1046, 678 |
| 56 | " | =N-OH | " | " | 1 | Schmp. 137-139°C |

| Nr. | Ar | X | $R^1$ | $R^2$ | n | Phys. Konstante oder IR-Spektr. [$cm^{-1}$] (Film) |
|---|---|---|---|---|---|---|
| 57 | $2,4-Cl_2C_6H_3-$ | $Cl-CH_2CH_2-\overset{O}{\overset{\|\|}{C}}-O-N=$ | $-CH_3$ | $n-C_4H_9$ | 1 | Harz |
| 58 | " | $CH_3CH_2\overset{O}{\overset{\|\|}{C}}-O-N=$ | " | " | 1 | Harz |
| 59 | " | O | " | $i-C_4H_9$ | 1 | 2961, 2938, 1707<br>1593, 1502, 1475<br>1390, 1276, 1137<br>1103, 1047, 835 |
| 60 | " | $=N-OH$ | " | " | 1 | Schmp. 160-162°C |
| 61 | " | $CH_3-CH=CH-CH_2-O-N=$ | " | " | 1 | Harz |
| 62 | " | $CH_3CH_2\overset{O}{\overset{\|\|}{C}}-O-N=$ | " | " | 1 | Harz |
| 63 | $4-(CH_3)C_6H_4-$ | O | " | $-CH_3$ | 1 | Schmp. 84-86°C |
| 64 | " | $=N-OH$ | " | " | 1 | Schmp. 166-168°C |
| 65 | " | $CH_3-CH_2-\overset{O}{\overset{\|\|}{C}}-O-N=$ | " | " | 1 | 2970, 2932, 1775<br>1495, 1456, 1345<br>1270, 1135, 1108<br>1045, 880, 805 |
| 66 | " | O | $-C_2H_5$ | " | 1 | Harz |

| Nr. | Ar | X | $R^1$ | $R^2$ | n | Phys. Konstante oder IR-Spektr. [$cm^{-1}$] (Film) |
|---|---|---|---|---|---|---|
| 67 | $4-(CH_3)C_6H_4-$ | =N-OH | $-C_2H_5$ | $-CH_3$ | 1 | Schmp. 138-140°C (Isom. I) |
| 68 | " | =N-OH | " | " | 1 | Schmp. 177-178°C (Isom.-Gemisch) |
| 69 | " | O | $-C_3H_7-n$ | " | 1 | 2970, 2940, 1708 1505, 1440, 1368 1278, 1138, 1060 1035, 810, 678 |
| 70 | " | =N-OH | " | " | 1 | Schmp. 198-200°C (Isom. I) |
| 71 | " | =N-OH | " | " | 1 | Schmp. 117-120°C (Isom.-Gemisch) |
| 72 | " | O | $-C_6H_{13}-n$ | " | 1 | Harz |
| 73 | " | =N-OH | " | " | 1 | Harz |
| 74 | $4-(C_2H_5)C_6H_4-$ | O | $-CH_3$ | " | 1 | 2965, 2938, 1695 1596, 1495, 1430 1270, 1132, 1045 822, 760, 678 |
| 75 | " | =N-OH | " | " | 1 | Schmp. 118-120°C |
| 76 | " | $4-(CH_3)C_6H_4-CH_2-O-N=$ | " | " | 1 | 2964, 2935, 1516 1502, 1445, 1366 1275, 1695, 1592 1016, 898, 810 |

| Nr. | Ar | X | $R^1$ | $R^2$ | n | Phys. Konstante oder IR-Spektr. [$cm^{-1}$] (Film) |
|---|---|---|---|---|---|---|
| 77 | 4-(tert.-$C_4H_9$)$C_6H_4$- | O | $-CH_3$ | $-CH_3$ | 1 | 2955, 1695, 1592 1492, 1355, 1266 1128, 1040, 949 873, 818, 675 |
| 78 | " | =N-OH | " | " | 1 | Schmp. 144-150°C |
| 79 | 4-($NO_2$)$C_6H_4$- | O | " | " | 1 | Schmp. 204-206°C (Isom. E) |
| 80 | " | O | " | " | 1 | Schmp. 103-105°C (Isom. Z) |
| 81 | " | =N-OH | " | " | 1 | Schmp. 172-174°C |
| 82 | 4-(CN)$C_6H_4$- | O | " | " | 1 | Harz |
| 83 | " | =N-OH | " | " | 1 | Harz |
| 84 | 2,4-($CH_3$)$_2C_6H_3$- | O | " | " | 1 | Schmp. 68-71°C |
| 85 | " | =N-OH | " | " | 1 | Schmp. 113-115°C |
| 86 | " | 4-(tert.-$C_4H_9$)$C_6H_4$-$CH_2$-O-N= | " | " | 1 | 2963, 1502, 1465 1445, 1364, 1275 1140, 1052, 1015 969, 889, 676 |
| 87 | 4-($CF_3$)$C_6H_4$- | O | " | " | 1 | 2948, 1707, 1620 1503, 1325, 1277 1166, 1124, 1068 1049, 1..9, 822 677 |

| Nr. | Ar | X | $R^1$ | $R^2$ | n | Phys. Konstante oder IR-Spektr. [$cm^{-1}$] (Film) |
|---|---|---|---|---|---|---|
| 88 | $4-(CF_3)C_6H_4-$ | $=N-OH$ | $-CH_3$ | $-CH_3$ | 1 | Schmp. 162-166°C |
| 89 | | $CH_3CH_2\overset{\overset{O}{\|}}{C}-O-N=$ | " | " | 1 | 2988, 2945, 1782<br>1503, 1326, 1277<br>1165, 1126, 1069<br>1053, 1019, 895 |
| 90 | " | $C_2H_5-O-N=$ | " | " | 1 | |
| 91 | " | O | $-C_2H_5$ | " | 1 | |
| 92 | " | $=N-OH$ | " | " | 1 | |
| 93 | " | $CH_3CH_2\overset{\overset{O}{\|}}{C}-O-N=$ | " | " | 1 | |

Die neuen Verbindungen können praktisch alle Entwicklungsstadien einer Pflanze verschiedenartig beeinflussen und werden deshalb als Wachstumsregulatoren eingesetzt.

Die Wirkungsvielfalt der Pflanzenwachstumsregulatoren hängt ab vor allem

a) von der Pflanzenart und -sorte,

b) von dem Zeitpunkt der Applikation, bezogen auf das Entwicklungsstadium der Pflanze und von der Jahreszeit,

c) von dem Applikationsort und -verfahren (Samenbeize, Bodenbehandlung oder Blattapplikation),

d) von den geoklimatischen Faktoren, z. B. Sonnenschein, Dauer, Durchschnittstemperatur, Niederschlagsmenge,

e) von der Bodenbeschaffenheit (einschließlich Düngung),

f) von der Formulierung bzw. Anwendungsform des Wirkstoffs und schließlich,

g) von den angewendeten Konzentrationen der aktiven Substanz.

Aus der Reihe der verschiedenartigen Anwendungsmöglichkeiten der erfindungsgemäßen Pflanzenwachstumsregulatoren im Pflanzenanbau, in der Landwirtschaft und im Gartenbau, werden einige nachstehend erwähnt.

A. Mit den erfindungsgemäß verwendbaren Verbindungen läßt sich das vegetative Wachstum der Pflanzen stark hemmen, was sich insbesondere in einer Reduzierung des Längenwachstums äußert. Die behandelten Pflanzen weisen demgemäß einen gedrungenen Wuchs auf ; außerdem ist eine dunklere Blattfärbung zu beobachten.

Als vorteilhaft für die Praxis erweist sich z. B. die Verringerung des Grasbewuches an Straßenrändern, Kanalböschungen und auf Rasenflächen wie Park-, Sport- und Obstanlagen, Zierrasen und Flugplätzen, so daß der arbeits- und kostenaufwendige Rasenschnitt reduziert werden kann.

Von wirtschaftlichem Interesse hat auch die Erhöhung der Standfestigkeit von lageranfälligen Kulturen wie Getreide, Mais, Sonnenblumen und Soja. Die dabei verursachte Halmverkürzung und Halmverstärkung verringern oder beseitigen die Gefahr des « Lagerns » (des Umknickens) von Pflanzen unter ungünstigen Witterungsbedingungen vor der Ernte.

Wichtig ist auch die Anwendung von Wachstumsregulatoren zur Hemmung des Längenwachstums und zur zeitlichen Veränderung des Reifeverlaufs bei Baumwolle. Damit wird ein vollständig mechanisiertes Beernten dieser wichtigen Kulturpflanze ermöglicht.

Durch Anwendung von Wachstumsregulatoren kann auch die seitliche Verzweigung der Pflanzen vermehrt oder gehemmt werden. Daran besteht Interesse, wenn z. B. bei Tabakpflanzen die Ausbildung von Seitentrieben (Geiztrieben) zugunsten des Blattwachstums gehemmt werden soll.

Mit Wachstumshemmern läßt sich beispielsweise bei Winterraps auch die Frostresistenz erheblich erhöhen. Dabei werden einerseits das Längenwachstum und die Entwicklung einer zu üppigen (und dadurch besonders frostanfälligen) Blatt- bzw. Pflanzenmasse gehemmt. Andererseits werden die jungen Rapspflanzen nach der Aussaat und vor dem Einsetzen der Winterfröste trotz günstiger Wachstumsbedingungen im vegetativen Entwicklungsstadium zurückgehalten. Dadurch wird auch die Frostgefährdung solcher Pflanzen beseitigt, die zum vorzeitigen Abbau der Blühhemmung und zum Übergang in die generativen Phase neigen. Auch bei anderen Kulturen, z. B. Wintergetreide ist es vorteilhaft, wenn die Bestände durch Behandlung mit erfindungsgemäßen Verbindungen im Herbst zwar gut bestockt werden, aber nicht zu üppig in den Winter hineingehen. Dadurch kann der erhöhten Frostempfindlichkeit und — wegen der relativ geringen Blatt- bzw. Pflanzenmasse — dem Befall mit verschiedenen Krankheiten (z. B. Pilzkrankheit) vorgebeugt werden. Die Hemmung des vegetativen Wachstums ermöglicht außerdem bei vielen Kulturpflazen eine dichtere Bepflanzung des Bodens, so daß ein Mehrertrag bezogen auf die Bodenfläche erzielt werden kann.

B. Mit den neuen Wirkstoffen lassen sich Mehrerträge sowohl an Pflanzenteilen als auch an Pflanzeninhaltsstoffen erzielen. So ist es beispielsweise möglich, das Wachstum größerer Mengen an Knospen, Blüten, Blättern, Früchten, Samenkörnern, Wurzeln und Knollen zu induzieren, den Gehalt an Zucker in Zuckerrüben, Zuckerrohr sowie Zitrusfrüchten zu erhöhen, den Proteingehalt in Getreide oder Soja zu steigern oder Gummibäume zum vermehrten Latexfluß zu stimulieren.

Dabei können die neuen Stoffe Ertragssteigerungen durch Eingriffe in den pflanzlichen Stoffwechsel bzw. durch Förderung oder Hemmung des vegetativen und/oder des generativen Wachstums verursachen.

C. Mit Pflanzenwachstumsregulatoren lassen sich schließlich sowohl eine Verkürzung bzw. Verlängerung der Wachstumsstadien als auch eine Beschleunigung bzw. Verzögerung der Reife der geernteten Pflanzenteile vor oder nach der Ernte erreichen.

Von wirtschaftlichem Interesse ist beispielsweise die Ernteerleichterung, die durch das zeitlich konzentrierte Abfallen oder Vermindern der Haftfestigkeit am Baum bei Zitrusfrüchten, Oliven oder bei anderen Arten und Sorten von Kern-, Stein- und Schalenobst ermöglicht wird. Derselbe Mechanismus,

das heißt die Förderung der Ausbildung von Trenngewebe zwischen Frucht- bzw. Blatt und Sproßteil der Pflanze ist auch für ein gut kontrollierbares Entblättern der Bäume wesentlich.

Die Wirkung der neuen Verbindungen ist besser als bei bekannten Wachstumsregulatoren. Die Wirkung zeigt sich sowohl bei Monokotylen, z. B. Getreide wie Weizen, Gerste, Roggen, Hafer und Reis oder Mais oder Gräsern als auch insbesondere bei Dikotylen (z. B. Sonnenblumen, Tomaten, Erdnüssen, Reben, Baumwolle, Raps und vor allem Soja) und verschiedenen Zierpflanzen wie Chrysanthemen, Poinsettien und Hibiskus.

Die erfindungsgemäßen Wirkstoffe können den Kulturpflanzen sowohl vom Samen her (als Saatgutbeizmittel) als auch über den Boden, d. h. durch die Wurzel sowie — besonders bevorzugt — durch Spritzung über das Blatt zugeführt werden.

Infolge der hohen Pflanzenverträglichkeit kann die Aufwandmenge stark variiert werden.

Bei der Saatgutbehandlung werden im allgemeinen Wirkstoffmengen von 0,001 bis 50 g je Kilogramm Saatgut, vorzugsweise 0,01 bis 10 g, benötigt.

Für die Blatt- und Bodenbehandlung sind im allgemeinen Gaben von 0,1 bis 12 kg/ha bevorzugt 0,25 bis 3 kg/ha als ausreichend zu betrachten.

Die erfindungsgemäßen Mittel können in Form üblicher Formulierungen angewendet werden wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z. B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel zugesetzt werden können. Als Hilfsstoffe zur Formulierung kommen im wesentlichen in Frage Lösungsmittel wie Aromaten (z. B. Xylol, Benzol), chlorierte Aromaten (z. B. Chlorbenzole), Paraffine (z. B. Erdölfraktionen), Alkohole (z. B. Methanol, Butanol), Amine (z. B. Ethanolamin), Dimethylformamid und Wasser ; feste Trägerstoffe wie natürliche Gesteinsmehle (z. B. Kaoline, Tonerden, Talkum, Kreide) und synthetische Gesteinsmehle (z. B. hochsdisperse Kieselsäure, Silikate) ; Emulgiermittel oder sonstige oberflächenaktive Mittel, wie nichtionogene und anionische Emulgatoren (z. B. Polyoxyethylen-Fettalkohol-Ether, Alkyl-sulfonate) und Dispergiermittel wie Lignin, Sulfitablaugen und Methylcellulose. Bevorzugt ist die Anwendung der erfindungsgemäßen Verbindungen in wäßriger Lösung gegebenenfalls unter Zusatz von mit Wasser mischbaren organischen Lösungsmitteln wie Methanol oder anderen niederen Alkoholen, Aceton, Dimethylformamid oder N-Methylpyrrolidin. Die Formulierungen enthalten im allgemeinen zwischen 0,1 und 95 Gewichtsprozent Wirkstoff, vorzugsweise zwischen 0,5 und 90.

Die Formulierungen bzw. die daraus hergestellten gebrauchsfertigen Zubereitungen wie Lösungen, Emulsionen, Suspensionen, Pulver, Stäube, Pasten oder Granulate werden in bekannter Weise angewendet, beispielsweise im Vorauflaufverfahren, im Nachauflaufverfahren oder als Beizmittel.

Beispiele für Formulierungen sind :

I 20 Gewichtsteile der Verbindung des Beispiels 2 werden in 3 Gewichtsteilen des Natriumsalzes der Diisobutylnaphthalin-sulfonsäure, 17 Gewichtsteilen des Natriumsalzes einer Ligninsulfonsäure aus einer Sulfitablauge und 60 Gewichtsteilen pulverförmigem Kieselsäuregel gut vermischt und in einer Hammermühle vermahlen. Durch feines Verteilen der Mischung in 20 000 Gewichtsteilen Wasser erhält man eine Spritzbrühe, die 0,1 Gew.% des Wirkstoffs enthält.

II 3 Gewichtsteile der Verbindung des Beispiels 3 werden mit 97 Gewichtsteilen feinteiligem Kaolin innig vermischt. Man erhält auf diese Weise ein Stäubemittel, das 3 Gew.% des Wirkstoffs enthält.

III 30 Gewichsteile der Verbindung des Beispiels 6 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

IV 40 Gewichtsteile der Verbindung des Beispiels 47 werden mit 10 Teilen Natriumsalz eines Phenolsulfonsäure-harnstoff-formaldehyd-Kondensats, 2 Teilen Kieselgel und 48 Teilen Wasser innig vermischt. Man erhält eine stabile wäßrige Dispersion. Durch Verdünnen mit 100 000 Gewichtsteilen Wasser erhält man eine wäßrige Dispersion, die 0,04 Gew.% Wirkstoff enthält.

V 20 Teile der Verbindung des Beispiels 52 werden mit 2 Teilen Calciumsalz der Dodecylbenzolsulfonsäure, 8 Teilen Fettalkohol-polyglykolether, 2 Teilen Natriumsalz eines Phenolsulfonsäure-harnstoff-formaldehyd-Kondensats und 68 Teilen eines paraffinischen Mineralöls innig vermischt. Man erhält eine stabile ölige Dispersion.

VI Man vermischt 90 Gewichtsteile der Verbindung des Beispiels 16 mit 10 Gewichtsteilen N-Methylpyrrolidon und erhält eine Lösung, die zur Anwendung in Form kleinster Tropfen geeignet ist.

VII 20 Gewichtsteile der Verbindung des Beispiels 88 werden in einer Mischung gelöst, die aus 80 Gewichtsteilen Xylol, 10 Gewichtsteilen des Anlagerungsproduktes von 8 bis 10 Mol Ethylenoxid an 1 Mol Ölsäure-N-monoethanolamid, 5 Gewichtsteilen Calciumsalz der Dodecylbenzolsulfonsäure und 5 Gewichtsteilen des Anlagerungsproduktes von 40 Mol Ethylenoxid an 1 Mol Ricinusöl besteht. Durch Ausgießen und feines Verteilen der Lösung in 100 000 Gewichtsteilen Wasser erhält man eine wäßrige Dispersion, die 0,02 Gew.% des Wirkstoffs enthält.

VIII 20 Gewichtsteile der Verbindung des Beispiels 89 werden in einer Mischung gelöst, die aus 40 Gewichtsteilen Cyclohexanon, 30 Gewichtsteilen Isobutanol, 20 Gewichtsteilen des Anlagerungsproduktes von 7 Mol Ethylenoxid an 1 Mol Isooctylphenol und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

IX 20 Gewichtsteile der Verbindung des Beispiels 87 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in 100 000 Gewichtsteilen Wasser erhält man eine wäßrige Dispersion, die 0,02 Gew.% des Wirkstoffs enthält.

Die erfindungsgemäßen Mittel können in diesen Aufwendungsformen auch zusammen mit anderen Wirkstoffen vorliegen, wie z. B. Herbiziden, Insektiziden, Wachstumsregulatoren und Fungiziden oder auch mit Düngemitteln vermischt und ausgebracht werden. Beim Vermischen mit Wachstumsregulatoren erhält man dabei in vielen Fällen eine Vergrößerung des Wirkungsspektrums. Bei einer Anzahl solcher Wachstumsregulatormischungen treten auch synergistische Effekte auf, d. h. die Wirksamkeit des Kombinationsproduktes ist größer als die addierten Wirksamkeiten der Einzelkomponenten.

Fungizide, die mit den erfindungsgemäßen Verbindungen kombiniert werden können, sind beispielsweise Dithiocarbamate und deren Derivate, wie
Ferridimethyldithiocarbamat,
Zinkdimethyldithiocarbamat,
Manganethylenbisdithiocarbamat,
Mangan-Zink-ethylendiamin-bis-dithiocarbamat,
Zinkethylenbisdithiocarbamat,
Tetramethylthiuramdisulfide,
Ammoniak-Komplex von Zink-(N,N-ethylen-bis-dithiocarbamat) und
N,N'-Polyethylen-bis-(thiocarbamoyl)-disulfid,
Zink-(N,N'-propylen-bis-dithiocarbamat),
Ammoniak-Komplex von Zinn-(N,N'-propylen-bis-dithiocarbamat) und
N,N'-Polypropylen-bis-(thiocarbamoyl)-disulfid ;
Nitrophenolderivate, wie
Dinitro-(1-methylheptyl)-phenylcrotonat,
2-sec-Butyl-4,6-dinitrophenyl-3,3-dimethylacrylat,
2-sec-Butyl-4,6-dinitrophenyl-isopropylcarbonat ;
heterocyclische Strukturen, wie
N-Trichlormethylthio-tetrahydrophthalimid,
N-Trichlormethylthio-phthalimid,
2-Heptadecyl-2-imidazol-acetat,
2,4-Dichlor-6-(o-chloranilino)-s-triazin,
0,0-Diethyl-phthalimidophosphonthionat,
5-Amino-1-(bis-(dimethylamino)-phosphinyl)-3-phenyl-1,2,4-triazol,
5-Ethoxy-3-trichlormethyl-1,2,4-thiadiazol,
2,3-Dicyano-1,4-dithiananthrachinon,
2-Thio-1,3-dithio-(4,5-b)-chinoxalin,
1-Butylcarbamoyl-2-benzimidazol-carbaminsäuremethylester,
2-Methoxycarbonylamino-benzimidazol,
2-Rhodanmethylthio-benzthiazol,
4-(2-Chlorphenylhydrazono)-3-methyl-5-isoxazolon, Pyridin-2-thiol-1-oxid,
8-Hydrochinolin bzw. dessen Kupfersalz,
2,3-Dihydro-5-carboxanilido-6-methyl-1,4-oxathiin-4,4-dioxid,
2,3-Dihydro-5-carboxanilido-6-methyl-1,4-oxathiin,
2-(Furyl-2))-benzimidazol,
Piperazin-1,4-diyl-bis-1-(2,2,2-trichlor-ethyl)-formamid,
2-Thiazolyl-(4))-benzimidazol,
5-Butyl-2-dimethylamino-4-hydroxy-6-methyl-pyrimidin,
Bis-(p-chlorphenyl)-3-pyridinmethanol,
1,2-Bis-(3-ethoxycarbonyl-2-thioureido)-benzol,
1,2-Bis-(3-methoxycarbonyl-2-thioureido)-benzol, und verschiedene Fungizide, wie
Dodecylguanidinacetat,
3-(2-(3,5-Dimethyl-2-oxycyclohexyl)-2-hydroxyethyl)-glutarimid,
Hexachlorbenzol,
N-Dichlorfluormethylthio-N,N'-dimethyl-N-phenyl-schwefelsäurediamid,
D,L-Methyl-N-(2,6-dimethyl-phenyl)-N-furyl(2)-alaninat,
D,L-N-(2,6-Dimethyl-phenyl)-N-(2'-methoxyacetyl)-alanin-methylester,

5-Nitro-isophthalsäure-di-isopropylester,
2,5-Dimethyl-furan-3-carbonsäureanilid,
2,5-Dimethyl-furan-3-carbonsäure-cyclohexylamid,
2-Methyl-benzoesäure-anilid,
1-(3,4-Dichloranilino)-1-formylamino-2,2,2-trichlorethan,
2,6-Dimethyl-N-tridecyl-morpholin bzw. dessen Salze,
2,6-Dimethyl-N-cyclododecyl-morpholin bzw. dessen Salze,
2,3-Dichlor-1,4-naphthochinon,
1,4-Dichlor-2,5-dimethoxybenzol,
p-Dimethylaminobenzol-diazinnatriumsulfonat,
1-Chlor-2-nitro-propan,

Polychlornitrobenzole, wie Pentachlornitrobenzol, Methylisocyanat, fungizide Antibiotika, wie Griseofulvin oder Kasugamycin, Tetrafluordichloraceton, 1-Phenylthiosemicarbazid, Bordeauxmischung, nickelhaltige Verbindungen und Schwefel.

Zur Bestimmung der wachstumsregulierenden Eigenschaft der Prüfsubstanzen wurden Testpflanzen auf ausreichend mit Nährstoffen versorgtem Kultursubstrat in Kunststoffgefäßen von ca. 12,5 cm Durchmesser angezogen.

Im Vorauflaufverfahren wurden die Testsubstanzen in wäßriger Aufbereitung am Tage der Einsaat auf das Saatbett gegossen.

Im Nachauflaufverfahren wurden die zu prüfenden Substanzen in wäßriger Zubereitung auf die Pflanzen gesprüht. Die beobachtete wachstumsregulierende Wirkung wurde bei Versuchsende durch Wuchshöhenmessung belegt. Die so gewonnenen Meßwerte wurden zur Wuchshöhe der unbehandelten Pflanzen in Relation gesetzt. Als Vergleich dienten Chlorcholinchlorid (A) und ein Triazolylketon (B), das aus der DE-OS 2 739 352, Beispiel 2 bekannt ist.

A (CCC)

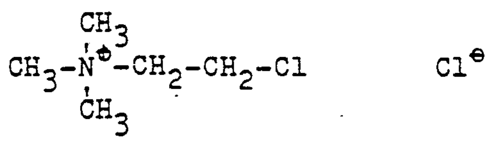

B (DE-OS 2 739 352 Beispiel 2)

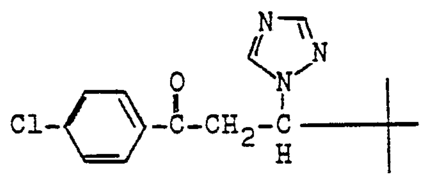

Gleichlaufend zur Reduzierung des Längenwachstums stieg die Farbintensität der Blätter an. Der erhöhte Chlorophyllgehalt läßt eine ebenfalls erhöhte Photosynthese und damit eine erhöhte Ertragsbildung erwarten.

Sommergerste, Sorte « Union », Vorauflaufverfahren

| Nr. d. chem. Beispiele | Konz. mg WS/Gef. | Wuchshöhen rel. |
|---|---|---|
| - | - | 100 |
| A (Vergleich) | 3 | 86,0 |
| | 12 | 78,0 |
| 12 | 3 | 85,5 |
| | 12 | 59,5 |
| 88 | 3 | 56,6 |
| | 12 | 49,1 |

Rasen — Nachauflaufverfahren

| Nr. d. chem. Beispiele | Konz. mg WS/Gef. | Wuchshöhen rel. |
|---|---|---|
| - | - | 100 |
| A (Vergleich) | 1,5<br>6,0 | 88,5<br>88,5 |
| 12 | 1,5<br>6,0 | 96,8<br>80,6 |
| 88 | 1,5<br>6,0 | 74,3<br>58,6 |

Sonnenblumen, Sorte « Sobrid » — Nachauflaufverfahren

| Nr. d. chem. Beispiele | Konz. mg WS/Gef. | Wuchshöhen rel. |
|---|---|---|
| - | - | 100 |
| A (Vergleich) | 1,5<br>6,0 | 89,5<br>84,4 |
| B (Vergleich) | 1,5<br>6,0 | 98,9<br>95,3 |
| 3 | 1,5<br>6,0 | 88,1<br>82,7 |
| 63 | 1,5<br>6,0 | 90,6<br>75,5 |
| 69 | 1,5<br>6,0 | 84,5<br>84,5 |
| 70 | 1,5<br>6,0 | 89,5<br>84,5 |
| 75 | 1,5<br>6,0 | 92,1<br>74,2 |
| 87 | 1,5<br>6,0 | 86,6<br>70,9 |
| 88 | 1,5<br>6,0 | 84,6<br>73,1 |

Sommerraps, Sorte « Petranova » — Vorauflaufverfahren

| Nr. d. chem. Beispiele | Konz. mg WS/Gef. | Wuchshöhen rel. |
|---|---|---|
| - | - | 100 |
| A (Vergleich) | 3<br>12 | 95,1<br>90,2 |
| B (Vergleich | 3<br>12 | 95,1<br>78,1 |
| 2 | 3<br>12 | 23,7<br>15,8 |
| 3 | 3<br>12 | 31,5<br>15,8 |

(Fortsetzung)

| Nr. d. chem. Beispiele | Konz. mg WS/Gef. | Wuchshöhen rel. |
|---|---|---|
| 6 | 3<br>12 | 35,5<br>23,7 |
| 9 | 3<br>12 | 74,8<br>58,4 |
| 18 | 3<br>12 | 35,5<br>11,9 |
| 19 | 3<br>12 | 75,6<br>48,8 |
| 23 | 3<br>12 | 72,0<br>38,1 |
| 39 | 3<br>12 | 86,9<br>70,5 |
| 40 | 3<br>12 | 61,1<br>43,7 |
| 43 | 3<br>12 | 51,2<br>39,4 |
| 46 | 3<br>12 | 74,8<br>55,2 |
| 47 | 3<br>12 | 67,0<br>47,3 |
| 50 | 3<br>12 | 84,7<br>63,6 |
| 64 | 3<br>12 | 35,5<br>21,7 |
| 65 | 3<br>12 | 82,5<br>51,5 |
| 67 | 3<br>12 | 67,8<br>50,8 |

Forts. Sommerraps, Sorte « Petranova » — Vorauflaufverfahren

| Nr. d. chem. Beispiele | Konz. mg WS/Gef. | Wuchshöhen rel. |
|---|---|---|
| 68 | 3<br>12 | 82,6<br>72,0 |
| 75 | 3<br>12 | 77,1<br>56,1 |
| 81 | 3<br>12 | 51,2<br>31,5 |
| 84 | 3<br>12 | 91,1<br>67,8 |
| 85 | 3<br>12 | 89,0<br>67,8 |
| 87 | 3<br>12 | 87,8<br>73,2 |
| 88 | 3<br>12 | 13,6<br>13,6 |

Sommerraps, Sorte « Petranova » — Nachauflaufverfahren

| Nr. d. chem. Beispiele | Konz. mg WS/Gef. | Wuchshöhen rel. |
|---|---|---|
| - | - | 100 |
| A (Vergleich) | 1,5 | 91,3 |
| | 6,0 | 84,5 |
| B (Vergleich) | 1,5 | 99,1 |
| | 6,0 | 94,8 |
| 2 | 1,5 | 73,4 |
| | 6,0 | 68,8 |
| 3 | 1,5 | 57,3 |
| | 6,0 | 48,5 |
| 18 | 1,5 | 77,1 |
| | 6,0 | 63,9 |
| 18 | 1,5 | 61,7 |
| | 6,0 | 55,1 |
| 36 | 1,5 | 90,1 |
| | 6,0 | 77,3 |
| 39 | 1,5 | 77,0 |
| | 6,0 | 64,1 |
| 40 | 1,5 | 72,4 |
| | 6,0 | 67,9 |
| 43 | 1,5 | 55,1 |
| | 6,0 | 48,5 |
| 46 | 1,5 | 59,5 |
| | 6,0 | 48,5 |
| 47 | 1,5 | 55,1 |
| | 6,0 | 50,7 |
| 50 | 1,5 | 73,0 |
| | 6,0 | 60,1 |
| 55 | 1,5 | 81,2 |
| | 6,0 | 66,3 |
| 56 | 1,5 | 63,3 |
| | 6,0 | 58,8 |
| 59 | 1,5 | 83,4 |
| | 6,0 | 64,1 |
| 64 | 1,5 | 66,1 |
| | 6,0 | 55,1 |
| 75 | 1,5 | 72,3 |
| | 6,0 | 68,2 |

Forts. Sommerraps, Sorte « Petranova » — Nachauflaufverfahren

| Nr. d. chem. Beispiele | Konz. mg WS/Gef. | Wuchshöhen rel. |
|---|---|---|
| 76 | 1,5 | 77,3 |
| | 6,0 | 60,4 |
| 81 | 1,5 | 52,9 |
| | 6,0 | 48,5 |
| 87 | 1,5 | 82,2 |
| | 6,0 | 73,1 |
| 88 | 1,5 | 70,0 |
| | 6,0 | 62,8 |

**Patentansprüche**

1. Triazolyl-ketonoxim und -dioxim der allgemeinen Formel I

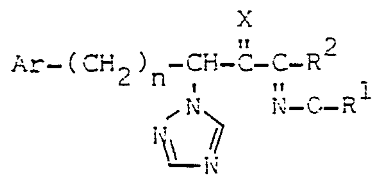

(I)

in der

Ar einen Arylrest aus der Gruppe Biphenylyl, Naphthyl oder Phenyl, der durch Halogenatome oder Trifluormethylreste oder durch Alkyl-, Alkoxy- oder Alkenylreste mit jeweils bis zu 5 C-Atomen und ferner durch einen Phenoxyrest substituiert sein kann, und

n 1 oder 2,

X Sauerstoff oder einen =N—OR$^3$-Rest bedeutet, wobei R$^3$ ein Wasserstoffatom, einen Alkyl-, Alkenyl- oder Alkinylrest mit jeweils bis zu 5 C-Atomen oder einen unsubstituierten oder durch Halogenatome, Trifluormethyl-, Alkyl- oder Alkoxyreste mit bis zu 4 C-Atomen substituierten Benzylrest oder einen —CO—R$^4$-Rest bedeutet, wobei R$^4$ einen Alkylrest mit bis zu 5 C-Atomen oder einen aromatischen Rest oder einen —NH—R$^5$-Rest bedeutet, wobei R$^5$ einen Alkylrest mit bis zu 4 C-Atomen oder einen aromatischen Rest bedeutet und

R$^1$ einen Alkylrest mit bis zu 8 C-Atomen, oder einen Phenylalkylrest bedeutet, und

R$^2$ einen Alkylrest mit bis zu 8 C-Atomen bedeutet.

2. Triazolyl-ketonoxime und -dioxime der Formel I gemäß Anspruch 1, in der

Ar einen Rest aus der Gruppe Biphenylyl, 1- und 2-Naphthyl, Phenyl, 2- und 4-Fluorphenyl, 3- und 4-Chlorphenyl, 4-Bromphenyl, 4-Methoxyphenyl, 4-Methoxyphenyl, 4-Ethoxyphenyl, 4-Propoxyphenyl, 4-Methylphenyl, 2,4-Dimethylphenyl, 4-Ethylphenyl, 4-Propylphenyl, 4-n-Butylphenyl, 4-Isobutylphenyl, 4-tert.-Butylphenyl, 4-Pentylphenyl, 4-Phenoxyphenyl, 3- und 4-Trifluormethylphenyl, 4-Allylphenyl, 3,4-Dichlorphenyl, 2,4-Dichlorphenyl und 2,6-Dichlorphenyl,

n 1 oder 2,

X Sauerstoff oder einen =N—OR$^3$-Rest, wobei

R$^3$ ein Wasserstoffatom oder einen Rest aus der Gruppe Methyl, Ethyl, 2-Methoxyethyl, n-Propyl, Isopropyl, n-Butyl, Isobutyl, Allyl, 2-Buten-1-yl, Pentenyl, Propargyl, Benzyl, 4-Fluorbenzyl, 4-Chlorbenzyl, 4-Trifluormethylbenzyl, 4-Brombenzyl, 4-Methylbenzyl, 4-tert.-Butylbenzyl, 2,4-Dichlorbenzyl, 3,4-Dichlorbenzyl, 2,6-Dichlorbenzyl oder einen CO—R$^4$-Rest, wobei

R$^4$ Methyl, Ethyl, n-Propyl, Isopropyl, Benzyl oder einen —NH—R$^5$-Rest,

R$^5$ Methyl, Ethyl, Propyl, n-Butyl, Phenyl oder 4-Chlorphenyl,

R$^1$ Methyl, Ethyl, 2-Methoxyethyl, n-Propyl, Isopropyl, n-Butyl, Isobutyl, n-Pentyl, Isopentyl, n-Hexyl, n-Octyl, Benzyl, 4-Chlorbenzyl, 2,4-Dichlorbenzyl, 4-Brombenzyl, 2-Phenylethyl,

R$^2$ Methyl, Ethyl, n-Propyl, Isopropyl, n-Butyl, Isobutyl, tert.-Butyl, n-Pentyl oder n-Heptyl bedeuten.

3. Triazolyl-ketonoxime der Formel II

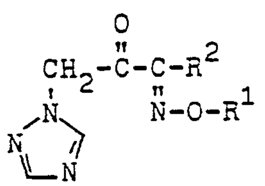

(II)

in der R$^1$ und R$^2$ die im Anspruch 1 bzw. 2 angegebene Bedeutung haben.

4. Die für Pflanzen verträglichen Salze und Metallkomplexe der Verbindungen I gemäß Anspruch 1 oder 2.

5. Verfahren zur Herstellung von Triazolyl-ketonoximen oder -dioximen der Formel I gemäß Anspruch 1 oder 2, dadurch gekennzeichnet, daß man Ketonoxime der Formel II

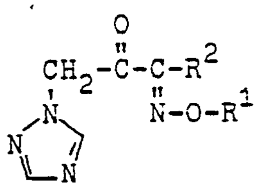

(II)

in der $R^1$ und $R^2$ die im Anspruch 1 bzw. 2 angegebenen Bedeutungen haben oder dessen Alkalienolat mit einem Arylalkylhalogenid der Formel III

$$Ar—(CH_2)_n—Y \qquad (III),$$

in der Ar und n die im Anspruch 1 bzw. 2 angegebenen Bedeutungen haben und Y Chlor, Brom oder Iod bedeutet, vorzugsweise in Gegenwart einer starken anorganischen oder organischen Base und gegebenenfalls unter Zusatz eines Reaktionsbeschleunigers und/oder eines Phasentransferkatalysators bei einer ausreichenden Temperatur bis zu 100 °C umsetzt und die so erhaltenen Verbindungen in den Fällen, in denen X Sauerstoff bedeutet, gegebenenfalls mit Hydroxylamin oder dessen Salzen umsetzt.

6. Verfahren zur Herstellung von Triazolyl-dioximen der Formel (I), in der $R^3$ von Wasserstoff verschieden ist, dadurch gekennzeichnet, daß man die gemäß Anspruch 5 erhaltenen Dioxime in an sich bekannter Weise mit einem Alkylierungsmittel der Formel

$$R^3—Y$$

in der $R^3$ die im Anspruch 1 angegebene Bedeutung hat und Y Chlor, Brom und Iod bedeutet, oder mit einem Säurechlorid der Formel $R^4—COCl$ oder mit einer Säureanhydrid der Formel $(R^4—CO)_2O$ oder ferner mit einem Isocyanat der Formel $R^5—N{=}C{=}O$ oder mit einem Carbamoylchlorid der Formel $R^5—NH—COCl$ in denen $R^4$ und $R^5$ die im Anspruch 1 angegebenen Bedeutungen haben, gegebenenfalls in Gegenwart eines Verdünnungsmittels und/oder einer anorganischen oder organischen Base und/oder eines Reaktionsbeschleunigers und/oder eines Phasentransferkatalysators bei einer ausreichenden Temperatur von bis zu 100 °C umsetzt.

7. Verfahren zur Herstellung von Triazolyl-dioximen der Formel I gemäß Anspruch 1, in denen X eine $=N—O—R^3$ -Gruppe bedeutet, dadurch gekennzeichnet, daß man Triazolyl-ketonoxime der Formel I gemäß Anspruch 1, in denen X Sauerstoff bedeutet, mit Hydroxylamin-Derivaten der Formel $H_2N—O—R^3$, in der $R^3$ die im Anspruch 1 angegebene Bedeutung hat, gegebenenfalls in Gegenwart eines Verdünnungsmittels umsetzt.

8. Mittel zur Regulierung des Pflanzenwachstums, enthaltend eine oder mehrere Verbindungen gemäß Anspruch 1, 2 oder 3.

9. Mittel zur Regulierung des Pflanzenwachstums, enthaltend eine oder mehrere Verbindungen gemäß Anspruch 1, 2 oder 3 und einen flüssigen oder festen Trägerstoff sowie gegebenenfalls eine odere mehrere oberflächenaktive Mittel.

10. Verfahren zur Regulierung des Pflanzenwachstums, dadurch gekennzeichnet, daß man eine oder mehrere Verbindungen gemäß Anspruch 1, oder 3 auf Pflanzen oder deren Lebensraum einwirken läßt.

11. Verfahren zur Herstellung von das Pflanzenwachstum regulierenden Mitteln, dadurch gekennzeichnet, daß man eine oder mehrere Verbindungen gemäß Anspruch 1, 2 oder 3 mit festen oder flüssigen Trägerstoffen sowie gegebenenfalls einem oder mehreren oberflächenaktiven Mitteln mischt.

## Claims

1. Triazolyl ketone-oximes and triazolyl dioximes of the general formula I

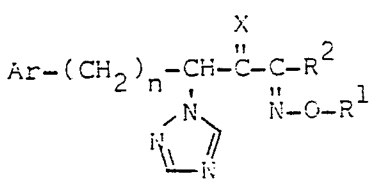

(I)

where

Ar is an aryl radical selected from the group consisting of biphenylyl, naphthyl and phenyl, which radical can be substituted by halogen or trifluoromethyl, by alkyl, alkoxy or alkenyl, each of up to 5 carbon atoms, or by phenoxy,

n is 1 or 2,

X is oxygen or $=N—OR^3$, $R^3$ being hydrogen, or alkyl, alkenyl or alkynyl, each of up to 5 carbon atoms, or benzyl which is unsubstituted or substituted by halogen or trifluoromethyl, or by alkyl or alkoxy, each of up to 4 carbon atoms, or is $—CO—R^4$, $R^4$ being alkyl of up to 5 carbon atoms, an aromatic radical or $—NH—R^5$, $R^5$ being alkyl of up to 4 carbon atoms or an aromatic radical,

$R^1$ is alkyl of up to 8 carbon atoms, or phenylalkyl, and

$R^2$ is alkyl of up to 8 carbon atoms.

2. Triazolyl ketone-oximes and triazolyl dioximes of the formula I as claimed in claim 1, where

Ar is a radical selected from the group consisting of biphenylyl, naphth-1-yl, naphth-2-yl, phenyl, 2- and 4-fluorophenyl, 3- and 4-chlorophenyl, 4-bromophenyl, 4-methoxyphenyl, 4-ethoxyphenyl, 4-propoxyphenyl, 4-methylphenyl, 2,4-dimethylphenyl, 4-ethylphenyl, 4-propylphenyl, 4-n-butylphenyl, 4-isobutylphenyl, 4-tert.-butylphenyl, 4-pentylphenyl, 4-phenoxyphenyl, 3- and 4-trifluoromethylphenyl, 4-allylphenyl, 3,4-dichlorophenyl, 2,4-dichlorophenyl and 2,6-dichlorophenyl,

n is 1 or 2,

X is oxygen or $=N—O—R^3$, where

$R^3$ is hydrogen or a radical selected from the group consisting of methyl, ethyl, 2-methoxyethyl, n-propyl, isopropyl, n-butyl, isobutyl, allyl, but-2-en-1-yl, pentenyl, propargyl, benzyl, 4-fluorobenzyl, 4-chlorobenzyl, 4-trifluoromethylbenzyl, 4-bromobenzyl, 4-methylbenzyl, 4-tert.-butylbenzyl, 2,4-dichlorobenzyl, 3,4-dichlorobenzyl, 2,6-dichlorobenzyl, or $CO—R^4$, where

$R^4$ is methyl, ethyl, n-propyl, isopropyl, benzyl, or $—NH—R^5$, where

$R^5$ is methyl, ethyl, propyl, n-butyl, phenyl or 4-chlorophenyl,

$R^1$ is methyl, ethyl, 2-methoxyethyl, n-propyl, isopropyl, n-butyl, isobutyl, n-pentyl, isopentyl, n-hexyl, n-octyl, benzyl, 4-chlorobenzyl, 2,4-dichlorobenzyl, 4-bromobenzyl or 2-phenylethyl, and

$R^2$ is methyl, ethyl, n-propyl, isopropyl, n-butyl, isobutyl, tert.-butyl, n-pentyl or n-heptyl.

3. Triazolyl ketone-oximes of the formula II

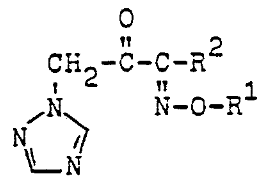

(II)

where $R^1$ and $R^2$ have the meanings given in claim 1 or 2.

4. Plant-tolerated salts and metal complexes of compounds I as claimed in claim 1 or 2.

5. A process for the manufacture of triazolyl ketone-oximes and triazolyl dioximes of the formula I as claimed in claim 1 or 2, wherein a ketone-oxime of the formula

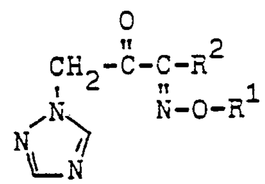

(II)

where $R^1$ and $R^2$ have the meanings given in claim 1 or 2, or its alkali metal enolate, is reacted with an arylalkylhalide of the formula III

$$Ar—(CH_2)_n—Y \qquad III,$$

where Ar and n have the meanings given in claim 1 or 2, and Y is chlorine, bromine or iodine, preferably in the presence of a strong organic or inorganic base and, if desired, with the addition of a reaction accelerator and/or a phase transfer catalyst at a sufficiently high temperature of up to 100 °C, and, if desired, the compounds thus obtained are, when X is oxygen, reacted with hydroxylamine or a salt thereof.

6. A process for the manufacture of triazolyl dioximes of the formula (I), where $R^3$ is not hydrogen, wherein the dioximes obtained as claimed in claim 5 are reacted in a conventional manner with an alkylating agent of the formula

$$R^3—Y,$$

where $R^3$ has the meanings given in claim 1, and Y is chlorine, bromine or iodine, or with an acid chloride of the formula $R^4—COCl$, or with an acid anhydride of the formula $(R^4—CO)_2O$, or with an isocyanate of the formula $R^5—N=C=O$, or with a carbamyl chloride of the formula $R^5—NH—COCl$, where $R^4$ and $R^5$ have the meanings given in claim 1, in the presence or absence of a diluent and/or an organic or inorganic base and/or a reaction accelerator and/or a phase transfer catalyst, at a sufficiently high temperature of up to 100 °C.

7. A process for the manufacture of triazolyl dioximes of the formula I as claimed in claim 1, where X is $=N—O—R^3$, wherein a triazolyl ketone-oxime of the formula I as claimed in claim 1, where X denotes oxygen, is reacted with a hydroxylamine derivative of the formula $H_2N—O—R^3$, $R^3$ having the meanings given in claim 1, in the presence or absence of a basic or acidic catalyst and in the presence or absence of a diluent.

8. An agent for regulating plant growth, containing one or more compounds as claimed in claim 1, 2 or 3.

9. An agent for regulating plant growth, containing one or more compounds as claimed in claim 1, 2 or 3, a solid or liquid carrier, and, if desired, one or more surfactants.

10. A process for regulating plant growth, wherein one or more compounds as claimed in claim 1, 2 or 3 are allowed to act on the plants or their habitat.

11. A process for manufacturing agents for regulating plant growth, wherein one or more compounds as claimed in claim 1, 2 or 3 are mixed with solid or liquid carriers and, if desired, with one or more surfactants.

## Revendications

1. Triazolyl-cétonoxime et -dioxime de formule générale

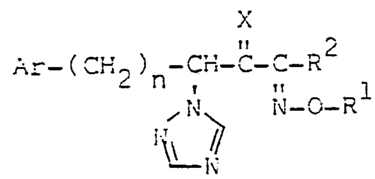

(I)

dans laquelle

Ar représente un reste aryle, du groupe de biphényle, naphtyle ou phényle, qui peut être substitué par des atomes d'halogène ou des restes trifluorométhyle ou par des restes alkyle, alcoxy ou alcényle ayant chacun jusqu'à 5 atomes C et, en outre, par un reste phénoxy, et

n est 1 ou 2,

X représente oxygène ou un reste $=N—OR^3$, $R^3$ représentant un atome d'hydrogène, un reste alkyle, alcényle ou alcinyle ayant chacun jusqu'à 5 atomes C, ou un reste benzyle, non substitué ou substitué par des atomes d'halogène, des restes trifluorométhyle, alkyle ou alcoxy ayant jusqu'à 4 atomes C, ou un reste $—CO—R^4$, $R^4$ représentant un reste alkyle ayant jusqu'à 5 atomes C ou un reste aromatique ou un reste $—NH—R^5$, $R^5$ représentant un reste alkyle ayant jusqu'à 4 atomes C ou un reste aromatique et

$R^1$ représente un reste alkyle ayant jusqu'à 8 atomes C ou un reste phénylealkyle et

$R^2$ un reste alkyle ayant 8 atomes C.

2. Triazolyl-cétonoxime et -dioxime de formule I selon la revendication 1, dans laquelle

Ar représente un reste du groupe biphényle, 1- et 2-naphthyle, phényle, 2- et 4-fluorophényle, 3- et 4-chlorophényle, 4-bromophényle, 4-méthoxyphényle, 4-éthoxyphényle, 4-propoxyphényle, 4-méthylphényle, 2,4-diméthylphényle, 4-éthylphényle, 4-propylphényle, 4-n-butylphényle, 4-isobutylphényle, 4-tert-butylphényle, 4-pentylphényle, 4-phénoxyphényle, 3- et 4-trifluorométhyle-phényle, 4-Allylphényle, 3-4-dichlorophényle, 2,4-dichlorophényle et 2,6-dichlorophényle,

n est 1 ou 2

X représente oxygène ou un reste $=N—OR^3$,

$R^3$ représentant un atome d'hydrogène ou un reste appartenant au groupe méthyle, éthyle, 2-méthoxyéthyle, n-propyle, isopropyle, n-butyle, isobutyle, allyle, 2-butène-1-yle, pentényle, propargyle, benzyle, 4-fluorobenzyle, 4-chlorobenzyle, 4-trifluorométhylbenzyle, 4-bromobenzyle, 4-méthylbenzyle, 4-tert-butylbenzyle, 2,4-dichlorobenzyle, 3,4-dichlorobenzyle, 2,6-dichlorobenzyle, ou un reste $CO—R^4$,

$R^4$ représentant méthyle, éthyle, n-propyle, isopropyle, benzyle ou un reste $—NH—R^5$,

$R^5$ méthyle, éthyle, propyle, n-butyle, phényle ou 4-chlorophényle,

$R^1$ représente méthyle, éthyle, 2-méthoxyéthyle, n-propyle, isopropyle, n-butyle, n-pentyle, isopentyle, n-hexyle, n-octyle, benzyle, 4-chlorobenzyle, 2,4-dichlorobenzyle, 4-bromobenzyle, 2-phényléthyle,

$R^2$ représente méthyle, éthyle, n-propyle, isopropyle, n-butyle, isobutyle, tert-butyle, n-pentyle ou n-heptyle.

3. Triazolyl-cétonoximes de formule II

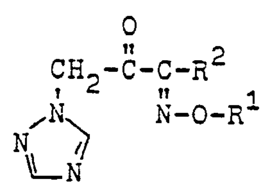

(II)

dans laquelle $R^1$ et $R^2$ ont la signification donnée dans la revendication 1 ou 2.

4. Les sels et complexes métalliques des composés I selon la revendication 1 ou 2, acceptables pour les plantes.

5. Procédé de préparation de triazolyl-cétonoximes ou- dioximes de formule I selon la revendication 1 ou 2, caractérisé par le fait que l'on fait réagir, à une température suffisante, jusqu'à 100 °C, de

préférence en présence d'une base forte organique ou inorganique et, éventuellement, avec addition d'un accélérateur de réaction et/ou d'un catalyseur de transfert de phases, des cétonoximes de formule

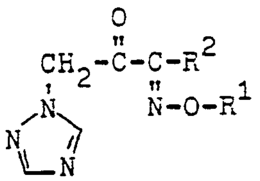

(II)

dans laquelle $R^1$ et $R^2$ ont les significations données dans la revendication 1 ou 2, ou leur énolate alcalin, avec un halogénure d'arylalkyle de formule III

$$Ar—(CH_2)_n—Y$$

dans laquelle Ar et n ont les significations données dans la revendication 1 ou 2, et Y représente chlore, ou iode, et dans les cas où X représente oxygène, on fait réagir les composés obtenus, éventuellement avec de l'hydroxylamine ou ses sels.

6. Procédé de préparation de triazolyl-dioximes de formule (I), dans laquelle $R^3$ est différent de hydrogène, caractérisé par le fait que l'on fait réagir, à une température suffisante, jusqu'à 100 °C, de préférence en présence d'une base forte organique ou inorganique et/ou d'un accélérateur de réaction et/ou d'un catalyseur de transfert de phases les dioximes obtenues selon la revendication 5, de manière usuelle, avec un agent d'alkylation de formule

$$R^3—Y$$

dans laquelle $R^3$ a la signification donnée dans la revendication 1 et Y représente chlore, brome et iode, ou avec un chlorure d'acide de formule $R^4—COCl$ ou avec un anhydride d'acide de formule $(R^4—CO)_2$ ou encore avec un isocyanate de formule $R^5—N=C=O$ ou avec un chlorure de carbamoyle de formule $R^5—NH—COCl$, dans laquelle $R^4$ et $R^5$ ont les significations données dans la revendication 1.

7. Procédé de préparation de triazolyl-dioximes de formule I selon la revendication 1, dans laquelle X représente un groupe $=N—O—R^3$, caractérisé par le fait que l'on fait réagir, éventuellement en présence d'un catalyseur basique ou acide et éventuellement en présence d'un diluant, des triazolyl-cétonoximes de formule I selon la revendication 1, dans laquelle X représente oxygène, avec des dérivés d'hydroxylamine de formule $H_2N—O—R^3$, dans laquelle $R^3$ a la signification donnée dans la revendication 1.

8. Moyen de régularisation de la croissance des plantes, contenant un ou plusieurs composés selon la revendication 1, 2 ou 3.

9. Moyen de régularisation de la croissance des plantes, contenant un ou plusieurs composés selon la revendication 1, 2 ou 3 et un support liquide ou solide, ainsi qu'éventuellement un ou plusieurs agents tensio-actifs.

10. Procédé de régularisation de la croissance des plantes, caractérisé par le fait que l'on fait agir sur les plantes ou leur biotrope un ou plusieurs composés selon la revendication 1, 2 ou 3.

11. Procédé de préparation d'agents de régularisation de la croissance des plantes, caractérisé par le fait que l'on mélange un ou plusieurs composés selon la revendication 1, 2 ou 3 avec des supports solides ou liquides, ainsi qu'éventuellement un ou plusieurs agents tensio-actifs.